Europäisches Patentamt

European Patent Office

Office européen des brevets

(11) Publication number: **0 212 465**
A2

## (12) EUROPEAN PATENT APPLICATION

(21) Application number: **86110915.5**

(22) Date of filing: **07.08.86**

(51) Int. Cl.⁴: **C 07 K 15/06,** C 12 N 5/00,
C 12 Q 1/00, C 07 K 3/02

(30) Priority: **14.08.85 US 765714**

(43) Date of publication of application: **04.03.87**
**Bulletin 87/10**

(84) Designated Contracting States: **AT BE CH DE FR GB IT LI
LU NL SE**

(71) Applicant: **Sloan-Kettering Institute For Cancer
Research, 1275 York Avenue, New York New
York 10021 (US)**

(72) Inventor: **Eisinger, Magdalena, 30 Pine Terrace,
Demarest New Jersey 07627 (US)**
Inventor: **Ogata, Shun-Ichiro, 75 - 14 193rd Street, Fresh
Meadows New York 11366 (US)**
Inventor: **Marko, Olga, 427 Polly Ann, Paramus New
Jersey 07652 (US)**
Inventor: **Old, Lloyd J., 600 West End Avenue, New York
New York 10024 (US)**

(74) Representative: **Patentanwälte Schulze Horn und
Hoffmeister, Goldstrasse 36, D-4400 Münster (DE)**

(54) **Method for enhancement of growth of human cells in culture.**

(57) Physiologically active growth factors produced by melanoma and astrocytoma cell lines support continued proliferation of slow-growing cells such as melanocytes in the absence of other added compounds.

WI-38, a fibroblast cell line derived from human embryonic lung, was the most active source of melanocyte growth factors. No melanocyte growth promoting activity was found in extracts of cultured neuroblastoma, renal cancer, normal keratinocytes or renal epithelium. Nerve growth factor, epidermal growth factor, melanocyte stimulating hormone, transforming growth factor – Beta, and platelet-derived growth factor lacked growth promoting activity for melanocytes. The presence of melanocyte growth factors and TPA together resulted in the strongest mitogenic activity for slow-growing cells such as melanocytes, permitting the recovery (at 20 days) of 4 to 20 times more cells than in factor or TPA alone. A synergistic or augmented effect is seen with TPA plus melanocyte growth factor.

This work was performed under a United States government grant PHS Grant Number 1 R01 CA 32152 from the National Cancer Institute. Therefore, the United States Government has certain rights in the invention.

## Summary

Cell extracts from various human cell sources provides a growth factor for certain cell lines especially slow-growing cell lines such as melanocytes.

## Description

Melanocytes are the melanin pigment producing cells of the body, which in normal human skin represent a minor cell population that undergoes mitosis only rarely (S.W. Downing, et al. (1974) J. Invest. Dermatol. 62, 450; T.B. Fitzpatrick, (1963) Dermatol. Wochenschr. 147, 481; W.K. Jimbow et al, (1975) J. Cell Biol. 66, 663). Little is known about melanocyte growth regulation, owing mainly to difficulties in obtaining sufficient numbers of melanocytes for studies in vitro. We have shown previously (M. Eisinger, et al., (1982) Proc. Natl. Acad. Sci. U.S.A. 79, 2018 and the subject of U.S. patent application S.N. 469,854 filed February 25, 1983) that 12-0-tetradecanoyl

3

phorbol 13-acetate (TPA) fosters replication of melanocytes in vitro by permitting the preferential attachment of melanocytes from skin cell suspensions and by stimulating them to grow. This mitogenic activity of TPA can be potentiated by cholera toxin (M. Eisinger, et al. (1982) Supra) and isobutylmethylxanthine (R. Halaban, et al. (1984) In Vitro 20, 447). In contrast to melanocytes, human melanoma cells generally grow vigorously in vitro in the absence of TPA, suggesting that their independent growth might be associated with the production of melanocyte growth factor or factors. To pursue this idea, we began a search for factors produced by melanoma and other cell types that would permit growth of melanocytes in the absence of TPA. Bovine brain seems to contain a factor capable of stimulating melanocyte growth (L. Wilkins, et al. (1985) J. Cell. Physiol. 122:350; B.A. Gilchrest, et al. (1984) J. Invest. Dermatol. 83:370).

Melanocytes were isolated and cultured as described (M. Eisinger, et al. (1982) Supra). To avoid fibroblast contamination we trypsinized the cultures and purified them by immune rosetting and Percoll gradients (O. Marko, et al. (1982) Exp. Cell Res. 142:309 also the subject of U.S. patent application S.N. 469,854), or we treated them with geneticin (R. Halaban, et al. (1984) Supra). Cultures used for experiments were free of contaminating fibroblasts as shown by leucine-aminopeptidase staining (K. Wolf (1964) Arch. Klin. Exp. Dermatol. 218:446; M. Regnier, et al. (1973) Acta. dermatovener (Stockholm) 53: 241). Nonpigmented melanoma cell lines were selected for study to avoid the toxic effects of intermediate metabolites in melanin synthesis. In preliminary studies, we evaluated melanocyte growth stimulating activity in the supernatant fluids of four melanoma cell lines. As little or no activity was detected, we turned to other cell extracts.

Cell extracts as opposed to cellular supernatants were found to promote cell growth of cells in culture. We note this activity especially on slow-growing cells such as melanocytes. As known growth factors lacked melanocyte growth promoting activity, we assume that we are dealing with one or a family of new growth factors. Cell extracts appear to be a far richer source of these new factors than culture supernatants, and it will be important to extend this approach to the search for growth factors for other differentiated cell types. Although it is possible that combinations of known growth factors would reproduce he effects observed with extracts, experiments to test this have been negative. As melanomas and astrocytomas originate from neuroectodermal progenitor cells, production of melanocyte growth factors could be a property of selected cell types derived from this lineage.

- 4 -

0212465

With regard to the potent mitogenic effect of WI-38, fibroblasts are known to have both mesodermal and neuroectodermal origin. In addition, the fact that growth promoting activity could be extracted from skin fibroblasts raises the possibility that dermal fibroblasts are involved in the regulation of melanocytes, an idea which has been widely held. A broader range of normal and malignant cells are now being tested to determine the relation of cell type to factor production. Defining the nature and number of these melanocyte growth factors represents the next step. The factor from WI-38 appears to be an acid- and alkaline-stable, heat-labile proteinaceous material. 70% activity is retained in pH10 bicarbonate buffer at 4°C after 30 minutes while it is stable at pH3 in glycine-HCl buffer at 4°C after 30 minutes. The material demonstrates a molecular weight of 40,000 daltons by G-100 gel filtration. Some molecular aggregation of the factor was seen and some 10,000 MW units were observed in SDS as well. The material is anionic, binding to DEAE at PH7. These characteristics distinguish it from known growth factors.

The following examples are for illustrative purposes only and are not to limit the invention to the express examples shown. It will be obvious to those skilled in the art that other cells of neuroectodermal, ectodermal

- 5 -

or mesodermal origin can be employed to isolate cell extracts useful for cell growth. These naturally occuring factors can be utilized to accelerate and sustain the growth of other cells in culture, especially slow-growing cells such as melanocytes and other cells in general.

It is useful to establish primary melanomas in culture. These primary melanomas often grow very slowly in the beginning. It will also be useful to establish tissue cultures which more closely represent the profile of cell types found the tissue in vivo. Thus tissue culture can be established using the factor to enhance the growth of slow-growing cells present in normal or tumor tissue culture. In tissue culture without the factor, fast-growing cells outgrow the culture eliminating the slow growers, as for example in epidermal tissue culture where melanocytes are the slow growing cells and are outgrown by the fast growing keratinocytes in tissue culture.

This may be a method to treat pigmentary disorders as well such as vitalago. In such a method one would create an antibody to the factor to treat such disorders as melanoma or vitalogo.

It would also be obvious to search for a naturally occuring or chemical antagonist to the growth factor(s) to slow melanoma. This human derived material or factor should also be less immunogenic than animal factors.

## Example I

Tests were done with extracts derived from two cell lines of neuroectodermal origin-namely, SK-MEL-131 melanoma and $AO_2V_4$ astrocytoma- and from the embryonic lung fibroblast line WI-38. Cells were obtained from Sloan-Kettering Institute for Cancer Research: the $AO_2V_4$ clone of AO astrocytoma cell line from Dr. J. Shapiro, and the SK-N-Mc Neuroblastoma cell line from Dr. J.L. Biedler.

Melanocyte growth promoting activity of extracts of SK-MEL-131 melanoma, $AO_2V_4$ astrocytoma, and WI-38 fibroblasts was determined using the parameter of $[^3H]$Thymidine incorporation and melanocyte cell growth. Melanocytes were maintained in Eagle's minimal essential medium (MEM) with Earle's salts; 0.01 mM nonessential amino acids; and 2 mM L-glutamine containing penicillin (100 units/ml), streptomycin at 0.1 mg/ml, and Fungizone at 0.25 microgram/ml and 10% FCS (cMEM) with one of the following supplements: Experiments A and F controls: (no supplement), Experiments B and G: SK-MEL-131 extract (1/1000 dilution), Experiments C and H: $AO_2V_4$ extract (1/1000 dilution), experiments D and I. WI-38 extract (1/1000 dilution), Experiments E and J: 12-0-tetradecanoyl phorbol 13-acetate (TPA) (10 ng/ml).

Foreskin melanocyte cultures (designated MC 1217) were grown in cMEM, TPA (10 ng/ml), and cholera toxin ($10^{-8}$M) for 11 passages. They were then seeded in the same medium in tissue culture cluster plates (96-flat bottom wells) at $5 \times 10^3$ per well (A to E as above) or in tissue culture cluster plates (12 wells) at $2.5 \times 10^4$ (E to J as above). Twenty-four hours later the cultures were washed three times with MEM (not containing TPA and cholera toxin) and incubated for 4 hours. Cells were then fed at 3-day intervals with cMEM containing cell extracts or TPA. Triplicate cultures were labeled for 6 hours with [$^3$h]thymidine (5 microcurie/ml) at 72, 96, 120, 144 hours after initial addition of TPA or cell extracts. After labeling, the cells were washed three times with phosphate buffered saline (PBS), dislodged from the wells by trypsin-EDTA solution and counted in Hydrofluor using a scintillation counter. For evaluation of cell growth, trypsinized cells from triplicate cultures were counted at 5-day intervals. Standard error of the mean was less than 10% of the mean c.p.m. and cell counts for each point.

Cell extracts: For the preparation of cell extracts, cultured cells (T.E. Carey, et al. (1976) Proc. Natl. Acad. Sci. U.S.A. 73:3278; J.R. Shapiro, et al. (1981) Cancer Res. 41:2349; M. Pfreundschuh, et al. (1978) Proc.

Natl. Acad. Sci. U.S.A. 75:5122; J.G. Cairncross, et al., (1982) Proc. Natl. Acad. Sci. U.S.A. 79:5641; J.L. Biedler, et al. (1973) Cancer Res. 33:2643; R. Ueda, et al., (1979) J. Exp. Med. 150:564; L. Hayflick, et al., (1961) Exp. Cell Res. 25:585; M. Eisinger, et al., (1979) Proc. Natl. Acad. Sci. U.S.A. 76:5340) were grown to confluency in their respective medium in the presence of 10% FCS (fetal calf serum). Following a thorough wash with PBS, cells were rmeoved by scraping with a rubber policeman, pelleted at 180g for 10 minutes and suspended in PBS (1:1, by volume). All suspensions were sonicated twice for 15 seconds, diluted 1:10, and clarified by two-step centrifugation at 16,000g for 20 minutes and 150,000g for 45 minutes. Protein content was approximately 1.4 mg/ml as determined by the Lowry procedure (Lowry et al, (1951) J. Biol. Chem. 193:265). The supernatants were separated into aliquots and kept frozen at 70°C.

Melanocytes cultured in the absence of TPA showed minimal $[^3H]$thymidine incorporation and no cell growth. In the presence of SK-MEL-131, $AO_2V_4$ or WI-38 whole cell extracts, melanocytes showed active $[^3H]$thymidine incorporation and repeated rounds of cell division. Extracts of SK-MEL-131 or $AO_2V_4$ resulted in a six-fold increase in $[^3H]$thymidine incorporation (as compared to melanocytes grown in the absence of added extract or TPA).

*10*

Extracts of WI-38 resulted in a 20-fold increase in $[^3H]$thymidine incorporation. With regard to cell growth and division, melanocytes cultured in the absence of TPA generally round up and detach, and after 20 days, cultures are lost. The effect of cell extracts on melanocyte growth parallels their effect of $[^3H]$thymidine incorporation, with extracts of WI-38 exerting the strongest mitogenic activity for melanocytes. Titration experiments showed that extracts diluted 1/500 to 1/1000 gave optimal stimulation of melanocyte growth; activity could still be detected at dilutions of 1/10,000. Growth inhibition, most likely nonspecific, was found with extracts diluted less than 1/100. Eighty percent of the growth stimulating activity of WI-38 extracts was removed by adsorbtion with normal melanocytes.

## Example II

Table 1 summarizes the results of tests with 14 cell extracts according to example I. Melanocyte growth promoting activity was detected in extracts from three melanoma cell lines, a noncultured melanoma specimen, two of four astrocytoma cell lines, and fibroblasts cultured from lung and skin. Extracts from two renal carcinoma cell lines, a neuroblastoma cell line, and an embryonic kidney cell strain and cultured keratinocytes had no melanocyte growth promoting effect.

- 10 -

## Example III

The following known growth factors were also tested according to Example I: transforming growth factor-Beta (TGF-Beta), epidermal growth factor (EGF), platelet-derived growth factor (PDGF), melanocyte stimulating hormone (MSH), and nerve growth factor (NGF) (R.K. Assoian, et al. (1983) J. Biol. Chem. 258:7155). TGF-Beta and EGF were provided by Drs. R. Assoian, A. Roberts and M. Sporn. TGF-Beta was prepared as originally described (R.K. Assoian, et al., J. Biol. Chem. 258, 7155 [1983]) except that the removal of urea was accomplished by desalting on C18 HPLC. TFG-Beta was tested at a concentration of 0.1 ng/ml, 0.5 ng/ml and 1.0 ng/ml. EGF was tested at 3 ng/ml, 6 ng/ml, 20 ng/ml, 40 ng/ml. For tests of TFG-Beta combined with TGF-alpha the concentration of TGF-Beta was 0.1 ng/ml and EGF 3 ng/ml or 6 ng/ml. PDGF was tested at 1 and 2 units/ml, NGF at 10, 50 and 100 ng/ml (from Collaborative Research, Inc.) and alphaMSH (Sigma) at 10, 50, 100 and 1000 ng/ml.

No melanocyte growth promoting activity was detected in cultures supplemented with these factors either by [3H]thymidine incorporation or by increase in cell numbers.

/2

## Example IV

Exposure of melanocytes to the combined action of TPA and active cell extracts resulted in a synergistic effect on cell growth. To judge the influence of added TPA on melanocyte growth promoting activity of cell extracts, melanocytes (see below) were cultured for 20 days in cMEM with the following supplements. A: Control (no supplement). B:TPA B' : TPA + cholera toxin. C':SK-MEL-131 extract. C: SK-MEL-131 extract + TPA D':$AO_2V_4$ astrocytoma extract. D:$AO_2V_4$ astrocytoma extract + TPA. E':WI-38 fibroblast extract. E:WI-38 fibroblast extract + TPA. TPA was at 10 ng/ml, cholera toxin $10^{-8}$ M, cell extracts were diluted 1/1000.

Source of melanocytes: An epidermal cell suspension derived from a human foreskin (1290) was seeded into tissue culture cluster dishes (12 wells) (2 x $10^5$ cells/well) in cMEM and the factors listed above. Twenty-four hours later, all unattached cells were removed and each well was refed with the appropriate medium. Three days later the cells were trypsinized and seeded at approximately 1 x $10^4$ cells/well into tissue culture cluster dishes (6 wells). Melanocytes were allowed to attach and later geneticin at 100 micrograms/ml was added for an overnight treatment (R. Halaban, et al. (1984) Supra). Following treatment, cells were grown for 20 days, typsinized and counted. Contamination by fibroblasts was excluded by staining for leucine aminopeptidase positive cells.

When cultured in either TPA or extract for 20 days, there was a 4 fold increase in cells numbers in cultures with TPA, 8- to 30-fold increase in cultures with extracts. Addition of TPA and extracts together resulted in a 40-fold increase in the case of SK-MEL-131 extracts, 50-fold increase in the case of $AO_2V_4$ astrocytoma extracts, and 90-fold increase in the case of WI-38 extracts. Melanocytes grown under these various conditions showed distinguishing morphologic features (x200 magnification). Growth in TPA alone or with added cholera toxin resulted in melanocytes showing a prominent nucleus surrounded by a thin rim of cytoplasm and long dendritic processes (TPA 10 mg/ml). Melanocytes grown in extracts were triangular or spindle-shaped, with one or multiple dendrites at each ($AO_2V_4$ astrocytoma extract diluted 1/1000). Addition of TPA to such cultures caused cell elongation and extension of processes (TPA 10 ng/ml $^+AO_2V_4$ astrocytoma extract diluted 1/1000). Whereas melanocytes grown in TPA or extracts alone were contact inhibited, the combined action of TPA and extracts cause the cells to continue to replicate after reaching confluency. Melanocytes cultured in extracts of melanoma, astrocytoma, or WI-38 were more highly pigmented than melanocytes cultured in TPA.

## Example V

Extracts can also support the growth of melanocytes when seeded at low cell densities. Melanocytes ($1 \times 10^2$ per square centimeter) grew in the presence of extracts, either alone or in combination with TPA, whereas little or no growth was observed when TPA or TPA combined with cholera toxin was added. Factors in the extracts appear therefore to support the growth of individual melanocytes very efficiently and permit their cloning; this cannot be achieved with cultures grown in TPA or TPA and cholera toxin.

TABLE 1

Melanocyte growth promoting activity of cell extracts

| Cell Extracts | | Effect on Melanocytes | |
|---|---|---|---|
| Designation | Source | [$^3$H]Thymidine incorporation Stimulation Index* ± S.E.M. | Increase in Cell Number** |
| SK-MEL-131 | Melanoma | . 6.5 ± 0.30 | + |
| SK-MEL-170 | Melanoma | 3.6 ± 0.25 | + |
| SK-MEL-178 | Melanoma | 3.3 ± 0.26 | + |
| Tumor tissue | Melanoma | 4.1 ± 0.28 | + |
| AO$_2$V$_4$ | Astrocytoma | 8.3 ± 0.35 | + |
| SK-MG-8 | Astrocytoma | 3.9 ± 0.30 | + |
| SK-MG-17 | Astrocytoma | 1.7 ± 0.18 | - |
| SK-MG-12 | Astrocytoma | 1.2 ± 0.09 | - |
| SK-N-MC | Neuroblastoma | 2.6 ± 0.13 | - |
| SK-RC-42 | Kidney carcinoma | 1.8 ± 0.16 | - |
| SK-RC-28 | Kidney carcinoma | 1.4 ± 0.07 | - |
| WI-38 | Embryonic lung | 21.3 ± 2.72 | + |
| FsFb 1143 | Foreskin fibroblast | 6.7 ± 0.25 | + |
| HEKC | Embryo kidney | 1.4 ± 0.09 | - |
| 1223 EC | Epidermis | 0.8 ± 0.02 | - |

- 15 -

Legend to Table 1:

Triplicate cultures were labeled for 4 hours with [3H]thymidine at 72, 96, 120, 144 hours after the initial addition of cell extracts. The number of [3H]thymidine counts incorporated at each time interval was added together to obtain a total [3H]thymidine count. Comparable studies of [3H]thymidine incorporation were carried out with melanocytes grown in the absence of cell extracts.

\* Stimulation index is given as the ratio of:

$$\frac{\text{Total [}^3\text{H]thymidine counts of melanocytes grown in the presence of factors}}{\text{Total [}^3\text{H]thymidine counts of melanocytes grown in the absence of factors}}$$

S.E.M. = Standard Error of the Mean

See Example 1 for determination of cell growth

\*\* (-) indicates no increase in cell numbers
(+) indicates one or more population doublings in 20 days

What is Claimed:

17

1. Partially purified physiologically-active cell growth factors extracted from whole human cells.

2. Extract of claim 1 useful to promote growth of slow-growing cells.

3. Extract of claim 1 useful to promote growth of melanocytes.

4. Extract of claim 3 useful to promote growth of melanocytes in culture.

5. Extract of claim 3 useful to promote growth of melanocytes in a synergistic manner with TPA.

6. Extract of claim 2 useful to promote growth of slow-growing cells in culture.

7. Partially purified melanocyte growth factor.

8. The partially purified human-derived melanocyte growth factor of claim 7 wherein the factor is acid and alkaline stable, heat labile and has a molecular weight of approximately 40,000 daltons.

9. The factor of claim 7 extracted from transformed human neuro-ectodermal cells.

10. The factor of claim 7 human extracted from normal human fibroblasts.

11. Method for determination of a human melanocyte growth factor which comprises

   a) growing human melanocytes in culture in the presence of an appropriate growth-stimulatory factor;

   b) observing the presence or absence of the growth stimulatory factor;

   c) transferring the melanocytes to medium without the stimulatory growth factor for a period of time sufficient to slow melanocyte growth,

   d) adding appropriate amounts of a melanocyte stimulatory growth factor test substance to the melanocyte cell culture and,

e)     observing the presence or absence of the stimulation of growth activity of the melanocytes.

12.   Method of Claim 11 wherein tritiated thymidine is added along with the test substance to determine DNA synthesis as the determinant of cell growth.

13.   Method for the production of partially purified human melanocyte growth factor which comprises: disrupting whole homogeneous cell and removing particulate matter sufficient to cause the factor to remain in the extract supernatant.

14.   Method for the stimulation of melanocyte growth using human cell  extract which comprises adding partially purified human-derived melanocyte growth factor to cultures of melanocytes.

15.   Method of claim 14 wherein TPA is used together with melanocyte growth factor.